(19) Europäisches Patentamt
European Patent Office
Office européen des brevets

(11) Publication number: 0 219 609 B1

(12) # EUROPEAN PATENT SPECIFICATION

(45) Date of publication of patent specification: 16.01.91 Bulletin 91/03

(51) Int. Cl.[5]: C07C 1/24, C07C 11/10

(21) Application number: 86108536.3

(22) Date of filing: 07.04.83

(54) **Process for dehydrating 2-alcohols.**

(43) Date of publication of application: 29.04.87 Bulletin 87/18

(45) Publication of the grant of the patent: 16.01.91 Bulletin 91/03

(84) Designated Contracting States: AT BE CH DE FR GB IT LI LU NL SE

(56) References cited:
FR-A- 2 151 750
GB-A- 997 958
GB-A- 1 278 981
US-A- 3 947 347
US-A- 4 061 594
US-A- 4 258 218

(61) Publication number of the earlier application in accordance with Art. 76 EPC: 0091662

(73) Proprietor: PHILLIPS PETROLEUM COMPANY
5th and Keeler
Bartlesville Oklahoma 74004 (US)

(72) Inventor: Drake, Charles Alfred
Route 1 Box 206
Nowata Oklahoma 74048 (US)

(74) Representative: Dost, Wolfgang, Dr. rer. nat. Dipl.-Chem. et al
Patent- u. Rechtsanwälte Bardehle, Pagenberg, Dost, Altenburg Frohwitter & Partner Galileiplatz 1
D-8000 München 80 (DE)

EP 0 219 609 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the production of α-olefins by the catalytic dehydration of 2-alcohols.

It is well known that alcohols may be dehydrated to produce monoolefinic materials by passing the alcohols over the heated oxides of certain metals, such as aluminum oxide, thorium oxide, silicon dioxide, titanium oxide, magnesium oxide, tungsten oxide, chromium oxide, and zirconium oxide or mixtures thereof. The prior technical literature on the subject of this type of catalytic dehydration indicates that alumina, thoria and several of the other metal oxides are equivalents in their dehydrating effect and usually may be used interchangeably. Pines and Haag have reported in a paper published at page 2847 of volume 83 of the Journal of the American Chemical Society (1961) that α-olefins (terminally unsaturated olefins) may be obtained by dehydrating primary alcohols over an alumina catalyst. These workers further state that when 2-alcohols are dehydrated over the alumina catalyst, a mixture of internal olefin and α-olefin results, with the more stable internal olefin predominating over the α-olefin. Obtaining a dehydration product in which the α-olefin predominates significantly is a challenge since the internal olefin product is the thermodynamically favored product.

U.S. 3,283,027 discloses that thorium oxide and a number of other metal oxides including cerium oxide and other oxides of the rare earths possess the capability of catalyzing the selective dehydration of 2-alcohols to α-olefins.

Such catalysts have been found to have several disadvantages. For example, thorium oxide is a very insoluble material which makes it a difficult material to apply to a support. Further, when thorium oxide was applied to glass bead support as shown in Example 4 of that patent, it was noted that the catalyst was quite fragile and that thoria fell off the glass beads and caused reactor plugging. When the thorium oxide-glass bead catalyst was employed as a catalyst in the dehydration of 4-methyl-2-pentanol, it was noted that at atmospheric pressure the conversion was quite low even at high reaction temperature. In order to actually be commercially attractive, the dehydration catalyst should be durable and should give good conversion and selectivity at atmospheric pressure.

An object of the present invention is to provide a dehydration catalyst that is durable and that is capable of giving good conversion and selectivity to α-olefin even at atmospheric pressure.

Another object is to provide a method for the production of 4-methyl-1-pentene and 3-methyl-1-butene in various ratios from 4-methyl-2-pentanol and ethylene.

In accordance with the present invention, there is provided a process for the selective dehydration of 2-alcohols to α-olefins as defined in claim 1. Preferred embodiments are set forth in the depending claims.

In accordance with yet another embodiment of the present invention, there is provided a method for the production of 4-methyl-1-pentene and 3-methyl-1-butene by the dehydration of 4-methyl-2-pentanol over a dehydration catalyst and then contacting the resulting mixture of 4-methyl-1-pentene and 4-methyl-2-pentene with ethylene in the presence of a disproportionation catalyst under conditions sufficient to promote conversion of 4-methyl-2-pentene to a mixture of 3-methyl-1-butene and propylene, wherein said dehydration catalyst is selective to 4-methyl-1-pentene production when large yields of 4-methyl-1-pentene are desired or to 4-methyl-2-pentene when large yields of 3-methyl-1-butene are desired.

The support used in the preferred embodiment of the present invention can be any suitable support containing an oxide of aluminium which has a surface area no greater than 20 $m^2$/gm, preferably no greater than 5 $m^2$/gm as determined by the BET nitrogen adsorption technique. Included within the scope of the invention are thus the low surface area aluminas and combinations of aluminum oxide and silicon oxide having low surface area. The preferred supports are those comprising α-alumina, especially those having a surface area of no greater than 1 $m^2$/gm. In a particularly preferred embodiment, the support is treated with a basic compound of a metal of Groups I and II of the Periodic Table.

The catalytically active metal oxides that are deposited on the defined support are selected from the group consisting of thorium oxide, scandium oxide, yttrium oxide, and the oxides of the rare earth metals, cerium, praseodymium, neodymium, prometheum, samarium, europium, gadolinium, terbium, dysprosium, holmium, erbium, thulium, ytterbium, and lutetium. Typical examples include $Ce_2O_3$, $CeO_2$, $Dy_2O_3$, $Er_2O_3$, $Eu_2O_3$, EuO, $Gd_2O_3$, $Ho_2O$, $Lu_2O_3$, $Nd_2O_3$, $Pr_2O_3$, $PrO_2$, $Pr_6O_{11}$, $Sm_2O_3$, SmO, $Tb_2O_3$, $TbO_2$, $Tb_4O$, $Tm_2O_3$, $Yb_2O_3$, YbO, $Se_2O_3$, $Y_2O_3$, and $ThO_2$.

The catalyst is considered to be effective for the dehydration of any 2-alcohol that is capable of being dehydrated to an alpha olefin. Examples of such 2-alcohols are those of the formula

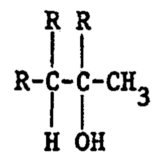

wherein at least one R is hydrocarbon in nature and wherein each R is individually selected from the group consisting of hydrogen, hydrocarbon radicals, and substituted hydrocarbon radicals wherein the substituents are selected from the keto, hydroxyl, alkoxy, and ester groups. Typically in order for 2-alcohols to be capable of being dehydrated to alpha olefins, aryl groups should be attached only at the number 4 position or a higher position, keto groups should be attached only at the number 5 position or a higher position, carbon-carbon olefinic or acetylenic unsaturation, hydroxyl groups, and ester groups should be attached only at the number 6 or higher position and preferably the number 7 or higher position. Obviously, the R on the 2 position carbon must be either H or a methyl group in order for the alcohol to be a 2-alcohol.

Typically, the hydrocarbyl radicals and the substituted hydrocarbyl radicals in the above formula would contain 1 to 17 carbon atoms. Generally, the preferred 2-alcohols are those in which the R of the number 2 position is hydrogen or a methyl group and at least one of the other R's is hydrogen and the remaining R group is selected from alkyl, cycloalkyl, or aralkyl groups having 1 to 17 carbon atoms, more preferably 1 to 10 carbon atoms.

The upper limit on the total carbon number of the suitable 2-alcohols is basically related to their ability to be vaporized under reasonable conditions. Thus extremely high boiling alcohols would be difficult if not impossible to use. Under very high vacuum, it is conceivable that alcohols having 30, 40, and even 50 carbon atoms per molecule could be employed. However, the preferred alcohols are those having no more than 20 carbon atoms per molecule.

A list of typical 2-alcohols meeting the requirements of the preceding general formula are disclosed in columns 3 and 4 of US-A-3,283,027.

The catalytic metal oxides can be deposited on the support in any suitable manner known in the art. The most common technique involves the deposition of a salt of the catalytic metal on the support followed by decomposition of the salt to an oxide of the catalytic metal. The currently preferred technique involves the use of a nitrate of the catalytic metal. An aqueous solution of the metal nitrate is poured over the support. After soaking for 30 minutes to an hour, the solution is concentrated by evaporation, and then the support containing the catalytic metal is dried in the presence of oxygen under such conditions that the metal salt is converted to the metal oxide. Typically, this involves heating in air at a temperature in the range of 300°C to 600°C.

The amount of catalytic metal oxide that can be employed in the present invention can vary over a wide range, with any catalytically effective amount being suitable. Typically, the total amount of the above defined catalytically active metal oxide is such that the total amount of the metal of catalytic metal oxide is in the range of 0.5 to 30 weight percent based on the weight of the support. More preferably, the total amount of the metal of the catalytic metal oxide is in the range of 2 to 25 weight percent based on the weight of the support.

A particularly preferred embodiment of the present invention involves the employment of a catalyst containing both cerium and thorium. In such catalysts, it is generally preferred for the weight percent of thorium to be in excess of the weight percent of the cerium. Typically for best results, the weight ratio of thorium to cerium should be in the range of 1/2 to 30/1, more preferably 1/1 to 10/1. In such catalysts particularly good results nave been obtained using about 5 to about 20 weight percent thorium based on the weight of the support.

As mentioned earlier, a particularly preferred embodiment of this invention involves the use of a support that has been treated with a basic compound of a metal of Groups I and II of the Periodic Table. The amount of basic compound that can be employed can vary over a wide range ; however, the conversion in the dehydration reaction appears to be inversely related to the level of basic compound. Accordingly, it is generally preferred that the amount of basic compound deposited on the support be no more than about 4 weight percent based on the weight of the support prior to the base treatment. Typical examples of basic compounds of Groups I and II include potassium hydroxide, cesium carbonate, sodium hydroxide, calcium hydroxide, and magnesium carbonate. The presently preferred basic compound is potassium hydroxide.

Since many of the preferred catalysts of the present invention employ a support that has been base treated, it is here noted that in this disclosure and the claims when a reference is made to the amount of a particular metal component, such as thorium, in terms of weight percent based on the weight of the support that is intended to refer to the weight of the support prior to any base treatment.

The basic compound can also be added to the support in any suitable manner. Presently, the preferred technique involves soaking the support with an aqueous solution of the basic compound prior to the addition of the catalytic metal.

In the dehydration of 2-alcohols with the catalysts of the present invention, the alcohol in a vapor state is passed in contact with the catalyst. The temperature at which the reaction is conducted is determined by a number of variables, the most important of which are the nature of the alcohol reactant, the residence time, and the extent of conversion desired. These variables can be readily ascertained for each alcohol by those skilled in the art by routine tests.

Generally, however, reaction temperature in the range of 330°C to 600°C are suitable, with temperatures in the range of 350°C to 450°C being preferred. The present catalysts can be employed at subatmospheric pressures and superatmospheric pressures, but are most desirable since even at atmospheric pressure they allow conversions and selectivities that were not attainable with the prior art catalysts.

A further understanding of the present invention and its advantages will be provided by the following examples. In the following examples, unless stated otherwise, if the aluminum oxide support is base treated, the general technique employed involved soaking 100 gm of the support with a 50 mL aqueous solution of a basic compound such as KOH for about 30 minutes. The liquid was poured off and the support washed three times with 50 to 100 mL aliquots of water.

The catalytically active metal was added to the support, after the base treatment, if a base treated support was employed. The general technique involved soaking 100 gms of the support with 50 mL of an aqueous solution of a nitrate of the catalytically active metal. After soaking for about 30 minutes, the solution was concentrated by evaporation, and then the support was dried in air at about 350°C for about 3 hours.

The dehydration reactions were carried out in a reactor tube 12-7mm (1/2") in diameter and 51 cm (20") long that was packed with 40 gm of the catalyst. Alcohol reactant was introduced into the reactor tubo at a rate of 36 mL/hr. A nitrogen sweep of 60 mL/min was employed.

Several different aluminum oxide containing supports are used in the following examples. The following table sets forth some of the characteristics of the supports :

| Support | Manufacturer | Composition, % | | | Surface Area, | |
|---|---|---|---|---|---|---|
| | | $Al_2O_3$ | $SiO_2$ | $Na_2O$ | $m^2/g$ | |
| R268* | Norton | 86.0 | 12.4 | - | 0.6 | Invention |
| T1370 | Girdler | 95.0 | .1-1.0 | .3-3.0 | 188.0 | Control |
| H151 | Alcoa | 98.0 | 1.2 | 0.7 | 324.0 | Control |
| 60-503 | Union Carbide (Linde) | 99.85 | 0.1 | 0.003 | 215 | Control |
| 61-501 | Union Carbide (Linde) | 99.75 | 0.15 | 0.01 | 230 | Control |

*A Phillips' identification number for Norton's SA-5123.

## Example I

A series of catalysts were prepared by treating various aluminium oxide supports with thorium nitrate. The catalyst were then employed in the dehydration of 4-methyl-2-pentanol. The results are summarized in Table I, wherein Runs 1 and 2 are according to the invention and Runs 3-11 are control runs.

Table I

| Run | Support | Th, Wt.%** | Temp,°C | Pressure | Alcohol Conversion,% | Olefin Selectivity,*% Total | 4-MP-1 | 4-MP-2 |
|---|---|---|---|---|---|---|---|---|
| 1 | R268 | 19.3 | 400 | Atm. | 89.6 | 82.4 | 95.8 | 3.9 |
| 2 | R268 | 19.3 | 410 | Atm. | 98.4 | 89.3 | 94.3 | 4.0 |
| 3 | T1370 | 9.7 | 290 | 207 kPa (30 psig) | 15.6 | n.d. | n.d. | n.d. |
| 4 | T1370 | 9.7 | 330 | " (30 psig) | 57.7 | n.d. | n.d. | n.d. |
| 5 | T1370 | 9.7 | 365 | " (30 psig) | 92.4 | 90.2 | 59.3 | 40.0 |
| 6 | T1370 | 19.3 | 290 | " (30 psig) | 8 | n.d. | n.d. | n.d. |
| 7 | T1370 | 19.3 | 330 | " (30 psig) | 40 | n.d. | n.d. | n.d. |
| 8 | T1370 | 19.3 | 350 | " (30 psig) | 92.1 | 82.4 | 46.2 | 53.5 |
| 9 | H151*** | 5.3 | 280 | Atm. | 100 | 99 | 31.3 | 67.2 |
| 10 | H151*** | 5.3 | 300 | Atm. | 99.5 | 98.5 | 30.5 | 67.1 |
| 11 | H151*** | 5.3 | 320 | Atm. | 98.6 | 96.6 | 27.8 | 66.9 |

*In this table and the following tables, Total is the percent of alcohol converted to olefin. The values under 4-MP-1 and 4-MP-2 refer to the percentage of the total olefin product represented by that particular olefin.

**Weight percent of Th as metal, based on the weight of the support.

***Thorium solution also contained 6 weight percent oxalic acid.

This example indicates that the effect of thorium on the dehydration reaction is much different for the support of the invention from the support of the control runs. The ratio of the 4-methyl-1-pentene to the 4-methyl-2-pentene is surprisingly higher for the low surface area support, i.e., Norton's R268 in Rūns/and 2.

## Example II

Another experiment was conducted using a series of catalysts prepared by treating the supports of invention Rūns/and 2 with potassium hydroxide and then with thorium nitrate. The effects of those catalysts on the dehydration of 4-methyl-2-pentanol are set forth in Table II.

Table II

| Support | Th, Wt.% | K, Wt.% | Temp, °C | Pressure | Alcohol Conversion,% | Olefin Selectivity,% Total | 4-MP-1 | 4-MP-2 |
|---|---|---|---|---|---|---|---|---|
| R268 | 19.3 | 2.8 | 370 | Atm. | 37.0 | 36.1 | 99.3 | 0.4 |
| R268 | 19.3 | 2.8 | 402 | Atm. | 69.7 | 67.1 | 99.2 | 0.4 |
| R268 | 19.3 | 2.8 | 412 | Atm. | 75.2 | 71.6 | 99.5 | 0.3 |
| R268 | 19.3 | 2.8 | 422 | Atm. | 85.5 | 81.3 | 99.6 | 0.3 |
| R268 | 19.3 | 2.8 | 434 | Atm. | 93.0 | 87.9 | 99.6 | 0.3 |
| R268 | 19.3 | 2.8 | 444 | Atm. | 95.6 | 90.2 | 99.4 | 0.3 |
| R268 | 19.3 | 2.8 | 446 | Atm. | 90.9 | 85.8 | 99.4 | 0.4 |
| R268 | 19.3 | 1.4 | 340 | 207kPa (30 psig) | 13.0 | n.d. | n.d. | n.d. |
| R268 | 19.3 | 1.4 | 370 | " (30 psig) | 29.8 | 28.8 | 99.0 | 0.5 |
| R268 | 19.3 | 1.4 | 397 | " (30 psig) | 51.5 | 48.8 | 99.2 | 0.4 |

A comparison of the results in Table II with Runs 1 and 2 of Table i reveals that the bas treatment of the supports results in a slight decrease in the overall conversion of the alcohol but an increase in the selectivity to the alpha olefin. Accordingly, by using the base treatment and slightly higher reaction temperatures, one can obtain higher yields of the alpha olefin than can be obtained at similar levels of alcohol conversion with a catalyst of thorium on a support which had not been base treated.

## Example III

Another catalyst was prepared by depositing thorium and cerium on the Norton R268 support. The thorium was deposited in an amount equal to 19.3 weight percent of the support. The cerium was deposited in an mount equal to 3.2 weight percent of the support. The effectiveness of this catalyst in the dehydration of 4-methyl-2-pentanol at atmospheric pressure is shown in Table III.

Table III

| Temp, °C | Alcohol Conversion, % | Olefin Selectivity, % Total | 4-MP-1 | 4-MP-2 |
|---|---|---|---|---|
| 370 | 65.1 | 61.8 | 98.8 | neg. |
| 400 | 92.1 | 86 | 98.7 | neg. |
| 410 | 96.7 | 91 | 98.9 | neg. |

A comparison of the results of Table III with those in Table I (Rūns 1 and 2) and Table II reveals that the alcohol conversion is slightly better for the Th-Ce catalyst than for the corresponding Th-base treated catalyst of Table II and the selectivity to 4-methyl-1-pentene is slightly better for the Th-Ce catalyst than for the corresponding non-base treated Th catalyst of Table 1.

## Example IV

In yet another series of runs, 19.3 weight percent thorium and 3.2 weight percent cerium were deposited on aluminum oxide supports that were pretreated with KOH. The results obtained when the catalysts were used to dehydrate 4-methyl-2-pentanol at atmospheric pressure are shown in Table IV.

Table IV

| Support | K, Wt.% | Temp, °C | Alcohol Conversion, % | Olefin Selectivity, % | | |
|---|---|---|---|---|---|---|
| | | | | Total | 4-MP-1 | 4-MP-2 |
| R268 | 2.8 | 370 | 63.6 | 61.8 | 99.3 | neg. |
| R268 | 2.8 | 400 | 89.2 | 86.2 | 99.6 | neg. |
| R268 | 2.8 | 410 | 95.4 | 91.6 | 99.6 | neg. |
| R268 | 1.4 | 370 | 64.9 | 62.6 | 99.6 | neg. |
| R268 | 1.4 | 390 | 84.9 | 80.8 | 99.6 | neg. |
| R268 | 1.4 | 410 | 96.7 | 91.1 | 99.6 | neg. |

A comparison of the results obtained with the Norton R268 support in this example with the results of Example III reveals that while the alcohol conversion is slightly lower for the base treated Th-Ce catalyst of this Example than for the Th-Ce catalyst of Example III, the selectivity to the alpha olefin is slightly better. Accordingly, where high yields of tHe α-olefin are desired, the preferred catalyst would be one having been treated with Th, Ce, and base.

Example V

Another series of catalysts were employed in the dehydration of 2-methyl-2-butanol. All the reactions were carried out at atmospheric pressure, employing a 36 mL/hr feed rate of 2-methyl-2-butanol, and 60 mL/min nitrogen flow rate. Reaction temperature, feed alcohol conversions and selectivities to 2-methyl-1-butene are set forth in Table V, wherein Rūns 1-6 are according to the invention and Rūns 7 and 8 are control runs. THe major reaction by-product is 2-methyL-2-butene.

Table V

| Run | Support | Th, Wt.% | Ce, Wt.% | K, Wt.% | Temp, °C | Alcohol Conversion, % | α-Olefin,%* |
|---|---|---|---|---|---|---|---|
| 1 | R268 | 19.3 | - | 0.7 | 350 | 99 | 64.3 |
| 2 | R268 | 19.3 | - | 0.3 | 350 | 99.1 | 57.9 |
| 3 | R268 | 10.0 | - | 7.0 | 350 | 6.8 | n.d. |
| 4 | R268 | 10.0 | - | - | 450 | 81.6 | 85.4 |
| 5 | R268 | 19.3 | 3.2 | 2.8 | 360 | 85.8 | 95.4 |
| 6 | R268 | 19.3 | 3.2 | 2.8 | 380 | 99.5 | 97.5 |
| 7 | 60-503 | - | - | 0.7 | 310 | 98.0 | 47.5 |
| 8 | 60-503 | - | - | 7.0 | 310 | 82.1 | 56.5 |

*Percentage of olefin product.

The above data reveals that the selectivity to α-olefin of the base treated Th-containing catalyst of the invention was better than that of the base treated of control rūns 7/8. The data further illustrates that the selectivity to the α-olefin is even better if Ce is employed in conjonction with the Th and base treatment.

The data presented in the foregoing examples and the prior art make it quite clear that in the dehydration of 4-methyl-2-pentanol, it is possible to obtain wide variations in the ratio of alpha olefin to internal olefin by the use of various types of catalysts. This fact can be applied to provide a process for the coproduction of selected ratios of 4-methyl-1-pentene and 3-methyl-1-butene, two specialty chemicals for which there is considerable demand. The process involves the dehydration of the 4-methyl-2-pentanol to obtain a mixture of 4-methyl-1-pentene and 4-methyl-2-pentene followed by the disproportionation of that olefin mixture with ethylene. Typically, it is desirable to remove substantially all the other dehydration reaction products and

unreacted alcohol from the mixture of 4-methyl-1-pentene and 4-methyl-2-pentene prior to subjecting that mixture to the disproportionation reaction. Any suitable conditions can be employed in the disproportionation reaction. Typical conditions are disclosed in U.S. Patents 3,457,320 and 3,658,932, the disclosures of which are incorporated herein by reference.

Thus, if one employs a dehydration catalyst of the type disclosed herein which gives an olefin product in which substantially all the olefin is 4-methyl-1-pentene, the coproduction process results mainly in 4-methyl-1-pentene. The coproduction process does however allow for the recovery of a more pure 4-methyl-1-pentene since it is much easier to separate 3-methyl-1-butene from 4-methyl-1-pentene by distillation than it is to separate 4-methyl-2-pentene from 4-methyl-1-pentene.

By employing a dehydration catalyst that gives an approximately 1 :1 ratio of 4-MP-1 to 4-MP-2, one can obtain a product from the metathesis reaction, a product having 4-MP-1, 3-MB-1, and propylene in about a 1 :1 :1 ratio.

By employing a dehydration catalyst that gives about a 2 :1 ratio of 4-MP-2 to 4-MP-1, one can obtain a product from the metathesis reaction having a 3-MB-1 to 4-MP-1 ratio of around 2/1.

Accordingly by simply varying the dehydration catalyst, it is possible to vary the relative yields of 3-MB-1 and 4-MP-1 over a wide range. A corresponding benefit is that the 4-MP-1 can be more readily recovered as a substantially pure product than it can from the reaction product of the dehydration reaction.

Example VI

Another series of catalysts were prepared using Th and Ce and other base treated low surface area aluminum oxide containing supports obtained from Norton. The supports and their relevant properties are summarized in Table VI.

Table VI

| Support | Composition, Wt. % $Al_2O_3$ | $SiO_2$ | Surface Area, $m^2/g$ |
|---|---|---|---|
| SA-5102 | 87 | 11 | 0.3 |
| SA-5158 | 99 | - | 0.7 |
| SA-3235 | 80 | 18 | 14 |

These catalysts, like the other previously discussed inventive catalysts, gave conversions of more than 90% with the 4-MP-1 product being the predominant olefin formed. The selectivity to 4-MP-1 was lower for the higher surface area support SA-3235 (14 $m^2/9$) coming, however, still within the scope of claim 1.

## Claims

1. A process for preparing α-olefins comprising contacting a 2-alcohol capable of being dehydrated to an α-olefin under dehydration conditions with a catalyst, characterized by using a catalyst comprising at least one catalytic metal oxide of metals having atomic numbers of 21, 39, 58-71, or 90, supported on an alumina-containing support, said support having a surface area no greater than 20 $m^2/g$.

2. The process of claim 1 characterized in that the total amount of said catalytic metal oxide is in the range of 0.5 to 30 weight percent based on the weight of the support.

3. The process of claim 1 or 2 characterized in that said at least one metal oxide is thorium oxide, or thorium oxide and cerium oxide.

4. The process of any of the preceding claims characterized in that the catalytic metal oxide consists essentially of thorium oxide and cerium oxide, the level of thorium is in the range of 5 to 20 weight percent based on the weight of the support, and the weight ratio of thorium to cerium is in the range of 1 :2 to 30 :1.

5. The process of any of the preceding claims characterized in that said support has been base treated.

6. The process of claim 5, characterized in that potassium hydroxide has been used in the base treatment.

7. The process of any of the preceding claims characterized in that said catalyst contains about 19 weight percent thorium metal based on the weight of said support and about 3 weight percent cerium metal based on the weight of said support.

8. The process of any of the preceding claims characterized in that said catalyst is prepared by soaking a support comprising alumina having a surface area no greater than 20 $m^2/g$ with an aqueous solution of potassium hydroxide, then washing the support with water, then washing the support with an aqueous solution of cerium nitrate and thorium nitrate, then evaporating the water, and then calcining at a temperature in the range of 300 to 600°C.

9. The process of any of the preceding claims, characterized in that said 2-alcohol has less than 20 carbon atoms per molecule.

10. The process of claim 9, characterized in that said 2-alcohol is of the formula

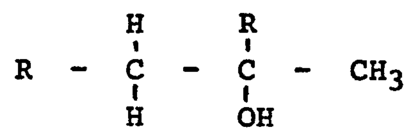

wherein the R in the 2 position is hydrogen or a methyl radical and the other R is an alkyl, cycloalkyl, or aralkyl group having 1 to 17 carbon atoms.

11. The process of claim 9 or 10 characterized in that said 2-alcohol is 4-methyl-2-pentanol or 2-methyl-2-butanol.

12. The process of claim 9 or 10 characterized in that after dehydration of 4-methyl-2-pentanol the resulting mixture of 4-methyl-1-pentene and 4-methyl-2-pentene is contacted with ethylene in the presence of a disproportionation catalyst to convert said 4-methyl-2-pentene to a mixture of 3-methyl-1-butene and propylene.

13. The process of claim 12, wherein the 4-methyl-1-pentene in the reaction product of the disproportionation reaction is separated from the remainder of the reaction product.

**Ansprüche**

1. Verfahren zur Herstellung von alpha-Olefinen, umfassend das Kontaktieren eines 2-Alkohols, der zur Dehydratisierung zu einem alpha-Olefin in der Lage ist, unter Dehydratisierungsbedingungen mit einem Katalysator, gekennzeichnet durch Verwendung eines Katalysators, der mindestens ein katalytisches Metalloxid von Metallen mit den Atomzahlen 21, 39, 58-71 oder 90 auf einem Aluminiumoxid enthaltenden Träger enthält, wobei der Träger eine Oberfläche von nicht mehr als 20 $m^2/g$ aufweist.

2. Verfahren nach Anspruch 1, dadurch gekennzeichnet, daß die Gesamtmenge des katalytischen Metalloxids im Bereich von 0,5-30 Gew.-%, bezogen auf das Gewicht des Trägers, liegt.

3. Verfahren nach Anspruch 1 oder 2, dadurch gekennzeichnet, daß es sich bei dem mindestens einen Metalloxid um Thoriumoxid oder Thoriumoxid und Ceroxid handelt.

4. Verfahren nach einem der vorstehenden Ansprüche, dadurch gekennzeichnet, daß das katalytische Metalloxid im wesentlichen aus Thoriumoxid und Ceroxid besteht, wobei der Anteil des Thoriums im Bereich von 5-20 Gew.-%, bezogen auf das Gewicht des Trägers, und das Gewichtsverhältnis von Thorium zu Cer im Bereich von 1 :2 bis 30 :1 liegen.

5. Verfahren nach einem der vorstehenden Ansprüche, dadurch gekennzeichnet, daß der Träger mit einer Base behandelt worden ist.

6. Verfahren nach Anspruch 5, dadurch gekennzeichnet, daß Kaliumhydroxid zur Basenbehandlung verwendet worden ist.

7. Verfahren nach einem der vorstehenden Ansprüche, dadurch gekennzeichnet, daß der Katalysator etwa 19 Gew.-% Thoriummetall, bezogen auf das Gewicht des Trägers, und etwa 3 Gew.-% Cermetall, bezogen auf das Gewicht des Trägers, enthält.

8. Verfahren nach einem der vorstehenden Ansprüche, dadurch gekennzeichnet, daß der Katalysator durch Einweichen eines Trägers mit einem Gehalt an Aluminiumoxid mit einer Oberfläche von nicht mehr als 20 $m^2/g$ mit einer wäßrigen Lösung von Kaliumhydroxid, anschließendes Waschen des Trägers mit Wasser, anschließendes Waschen des Trägers mit einer wäßrigen Lösung von Cernitrat und Thoriumnitrat, anschließendes Abdampfen des Wassers und anschließendes Calcinieren bei einer Temperatur im Bereich von 300-600°C hergestellt worden ist.

9. Verfahren nach einem der vorstehenden Ansprüche, dadurch gekennzeichnet, daß der 2-Alkohol weniger als 20 Kohlenstoffatome pro Molekül enthält.

10. Verfahren nach Anspruch 9, dadurch gekennzeichnet, daß der 2-ALkohol folgende Formel aufweist

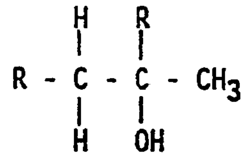

in der der Rest R in der 2-Stellung Wasserstoff oder eine Methylgruppe bedeutet und der andere Rest R einen Alkyl-, Cycloalkyl- oder Aralkylrest mit 1-17 Kohlenstoffatomen bedeutet.

11. Verfahren nach Anspruch 9 oder 10, dadurch gekennzeichnet, daß es sich bei dem 2-Alkohol um 4-Methyl-2-pentanol oder 2-Methyl-2-butanol handelt.

12. Verfahren nach Anspruch 9 oder 10, dadurch gekennzeichnet, daß nach der Dehydratisierung des 4-Methyl-2-pentanols das erhaltene Gemisch aus 4-Methyl-1-penten und 4-Methyl-2-penten mit Ethylen in Gegenwart eines Disproportionierungskatalysators in Kontakt gebracht wird, um das 4-Methyl-2-penten in ein Gemisch aus 3-Methyl-1-buten und Propylen überzuführen.

13. Verfahren nach Anspruch 12, wobei das 4-Methyl-1-penten im Reaktionsprodukt der Disproportionierungsreaktion vom Rest des Reaktionsprodukts abgetrennt wird.

**Revendications**

1. Procédé pour préparer des α-oléfines comprenant de mettre en contact un alcool 2 pouvant être déshydraté en une a-oléfine dans des conditions de déshydratation avec un catalyseur, caractérisé en ce qu'on utilise un catalyseur comprenant au moins un oxyde métallique catalytique de métaux ayant les numéros atomiques 21, 19, 58-71 ou 90, déposé sur un support contenant de l'alumine, ledit support ayant une surface ne dépassant pas 20 $m^2/g$.

2. Procédé selon la revendication 1, caractérisé en ce que la quantité totale dudit oxyde métallique catalytique se situe dans la gamme allant de 0,5 à 10 pour-cent en poids, exprimé par rapport au poids du support.

3. Procédé selon la revendication 1 ou 2, caractérisé en ce qu'au moins un desdits oxydes métalliques est l'oxyde de thorium ou l'oxyde de thorium avec de l'oxyde de cérium.

4. Procédé selon l'une quelconque des revendications précédentes, caractérisé en ce que l'oxyde métallique catalytique consiste essentiellement en oxyde de thorium et en oxyde de cérium, que le niveau de thorium se situe dans la gamme allant de 5 à 20 pour-cent en poids, exprimé par rapport au poids du support, et que le rapport pondéral thorium sur cérium se situe dans la gamme allant de 1 :2 à 10 :1.

5. Procédé selon l'une quelconque des revendications précédentes, caractérisé en ce que ledit support a subi un traitement basique.

6. Procédé selon la revendication 5, caractérisé en ce que de l'hydroxyde de potassium a été utilisé dans le traitement basique.

7. Procédé selon l'une quelconque des revendications précédentes, caractérisé en ce que ledit catalyseur contient environ 19 pour-cent en poids de métal thorium, exprimé par rapport au poids dudit support et environ 3 pour-cent de métal cérium, exprimé par rapport au poids dudit support.

8. Procédé selon l'une quelconque des revendications précédentes, caractérisé en ce que ledit catalyseur est préparé en faisant tremper un support comprenant de l'alumine ayant une surface ne dépassant pas 20 $m^2/g$ dans une solution aqueuse d'hydroxyde de potassium, puis en lavant le support avec de l'eau, en lavant le support avec une solution aqueuse de nitrate de cerium et de nitrate de thorium, puis en évaporant l'eau et enfin en calcinant à une température située dans la gamme allant de 100 à 600°C.

9. Procédé selon l'une quelconque des revendications précédentes, caractérisé en ce que ledit alcool 2 a moins de 20 atomes de carbone par molécule.

10. Procédé selon la revendication 9, caractérisé en ce que ledit alcool 2 a la formule :

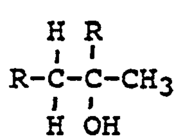

où le R en position 2 est un hydrogène ou un radical méthyle et l'autre R est un groupe alkyle, cycloalkyle ou aralkyle ayant de 1 à 17 atomes de carbone.

11. Procédé selon la revendication 9 ou 10, caractérisé en ce que ledit alcool 2 est le 4-méthyl-2-pentanol ou un 2-méthyl-2-butanol.

12. Procédé selon la revendication 9 ou 10, caractérisé en ce qu'après la déshydratation du 4-méthyl-2-pentanol, le mélange résultant de 4-méthyl-1-pentène et de 4-méthy1-2-pentène est mis en contact avec de l'éthylène en présence d'un catalyseur de disproportionnement pour convertir ledit 4-méthyl-2-pentène en un mélange de 3-méthyl-1-butène et de propylène.

13. Procédé selon la revendication 12, où le 4-méthyl-1-pentène dans le produit de réaction de la réaction de disproportionnement est séparé du reste du produit de réaction.